# FASCICULE DE BREVET EUROPEEN

(11) **EP 3 784 181 B1**
(45) Date de publication et mention de la délivrance du brevet: **25.05.2022**
(21) Numéro de dépôt: 19734844.4
(22) Date de dépôt: 25.04.2019
(51) Int. Cl.: A61F 9/008

(54) **SYSTEME DE PHOTOCOAGULATION LASER D'UNE RETINE**
SYSTEM ZUR LASERPHOTOKOAGULATION DER NETZHAUT
SYSTEM FOR LASER PHOTOCOAGULATION OF THE RETINA

(30) Priorité: 27.04.2018 FR 1853721
(43) Date de publication de la demande: 03.03.2021
(73) Titulaire: Quantel Medical, 63800 Cournon-d'Auvergne (FR); Office National d'Etudes et de Recherches Aérospatiales (ONERA), 91120 Palaiseau (FR)
(72) Inventeur: CHABRIER, Christian, 63670 La Roche Blanche (FR); MEIMON, Serge, 92322 Chatillon (FR); GAYOT, Patrice, 63800 Cournon D'Auvergne (FR); PETIT, Cyril, 91370 Verrieres Le Buisson (FR); BARACAL DE MECE, Pedro, 92200 Neuilly Sur Seine (FR); WASSMER, Benjamin, 63800 Cournon D'Auvergne (FR)
(74) Mandataire: Regimbeau
(86) Numéro de dépôt international: PCT/FR2019/050974
(87) Numéro de publication internationale: WO 2019/207253

(56) Documents cités:
- WO-A1-2014/013438
- US-A1- 2004 233 388

## Description

### DOMAINE TECHNIQUE DE L'INVENTION

La présente invention appartient au domaine de l'ophtalmologie. Plus précisément, l'invention concerne un système de photocoagulation laser d'une rétine.

L'œdème maculaire est une affection rétinienne assez fréquente se caractérisant par un d'un œdème de la région centrale de la rétine (macula) lié à des anomalies des petits vaisseaux sanguins, entraînant une baisse de l'acuité visuelle. L'œdème maculaire est la principale manifestation de la rétinopathie diabétique, qui est la cause principale de cécité avant 55 ans. Le traitement standard de ces œdèmes maculaires consiste à réaliser une photocoagulation de ces vaisseaux dans la zone centrale de la rétine à l'aide d'un laser. D'autres maladies peuvent également bénéficier de la photocoagulation de la macula, comme les occlusions veineuses rétiniennes.

Lors d'une photocoagulation, la cible thérapeutique du laser peut se situer au niveau de deux couches :
- la couche des vaisseaux sanguins rétiniens, dans le cas d'un œdème localisé. Dans ce cas, l'œdème est causé par une dilatation vasculaire appelée macroanévrysme sur un vaisseau rétinien identifié, et l'on procède à une photocoagulation directe du vaisseau impliqué ;
- l'épithélium pigmentaire rétinien, dans le cas d'un œdème diffus. Une solution consiste alors à stimuler la fonction d'absorption de l'épithélium pigmentaire, en le photocoagulant avec des impacts répétés.

Ces deux couches de la rétine sont séparées d'une centaine de microns, et sont entourés d'autres couches correspondant à des tissus fonctionnels qu'il faut s'efforcer de préserver, sous peine de provoquer une baisse de vision définitive. Jusqu'à présent, le chirurgien opérant le laser ne disposait que d'une visualisation bidimensionnelle de la rétine à la lampe à fente, vue de face, sur laquelle le chirurgien s'appuie pour localiser le point d'impact laser en surface de la rétine, et doser la puissance du laser : il est recommandé d'appliquer le laser jusqu'à observer un discret blanchiment à la lampe à fente. Il apparaît donc que dans les procédures chirurgicales actuelles, la focalisation et le dosage du laser sont très empiriques. De fait, les procédures de traitement standard sont assez peu reproductibles d'un praticien à l'autre.

En outre, pendant l'opération, l'œil est plus moins stabilisé avec un verre de contact maintenu sur l'œil, qui ne filtre que très partiellement les continuels mouvements involontaires de fixation de l'œil. Il en résulte que l'impact laser peut ne pas correspondre à la position visée sur l'image de fond d'œil obtenue par la lampe à fente.

Pour pallier ce manque de précision dans la localisation de l'impact laser, les systèmes actuels sont configurés pour générer un impact laser de grande taille, afin de s'assurer que la photocoagulation recouvre entièrement la zone à traiter. La grande taille de l'impact laser est obtenue par la délivrance d'un faisceau laser large, avec un diamètre latéral de la tache focale sur la rétine allant de 100 µm à 500 µm, et présentant une faible ouverture optique, avec une extension longitudinale (en profondeur) de la tache focale sur la rétine d'environ 300 µm.

Par ailleurs, un impact laser de grande taille permet également de pallier différentes aberrations oculaires réduisant la précision de la localisation de l'impact laser. En effet, un œil humain réel n'est pas strictement stigmatique, c'est-à-dire que l'image d'un point n'est pas un point rigoureusement net. Ces aberrations oculaires peuvent être statiques, les exemples les plus courants étant les défauts de vision corrigés par les lunettes (myopie, hypermétropie, astigmatisme, etc.). Les aberrations peuvent également être dynamiques, par exemple causées par des micro-accommodations du cristallin, l'écoulement du film lacrymal, et les mouvements oculaires. Ces aberrations se traduisent par une fonction d'étalement du point ("Point Spread Function" ou PSF en anglais) qui s'éloigne de celle d'un œil parfait théorique, et qui varie rapidement dans le temps.

La grande taille des impacts laser actuels n'est pas adaptée à la taille des zones à traiter. A titre d'exemple, les macro-anévrysmes présentent des tailles variant typiquement entre 100 µm et 300 µm, alors même que l'impact laser présente un diamètre d'environ 300 µm, sans compter la diffusion thermique se produisant autour de la zone d'impact laser. Il en résulte qu'apparaissent des lésions des tissus sains avoisinants la zone à traiter. Or, la couche des vaisseaux sanguins rétiniens et l'épithélium pigmentaire rétinien sont entourés de tissus fonctionnels dont l'altération peut entraîner une baisse de vision définitive.

### PRESENTATION DE L'INVENTION

L'invention a pour but de proposer un système de photocoagulation laser d'une rétine permettant une localisation précise de l'impact laser, tant en surface de la rétine qu'en profondeur, et confinant cet impact laser dans une zone restreinte, typiquement inférieure à 100 µm, afin de limiter les lésions occasionnées aux tissus sains au voisinage de la zone à traiter.

A cet effet, il est proposé un système de photocoagulation laser d'une rétine, comprenant :
- un laser de photocoagulation comprenant une source laser et une fibre optique de transport,
- un chemin optique reliant le laser de photocoagulation en amont à une sortie laser en aval destinée à être placée face à la rétine, un faisceau laser émis par le laser de photocoagulation étant destiné à se propager le long du chemin optique,
dans lequel la source laser est une source laser configurée pour émettre dans une longueur d'onde comprise entre 520 nm et 690 nm dans la fibre optique, et en ce que la fibre optique de transport présente un cœur avec un diamètre inférieur à 50 µm,
le système comprenant en outre :
- une optique adaptative positionnée dans le chemin optique et configurée pour modifier le front d'onde du faisceau laser se propageant dans le chemin optique afin de compenser des aberrations oculaires se produisant jusqu'à la rétine,
- une boucle de régulation en position commandant un premier actionneur positionné dans le chemin optique en aval de l'optique adaptative pour asservir en position la sortie laser vis-à-vis de la rétine à traiter,
- au moins un imageur configuré pour acquérir une image de la rétine dérivée du chemin optique.

Le système est avantageusement complété par les caractéristiques suivantes, prises seules ou en une quelconque de leur combinaison techniquement possible :
- un diamètre du faisceau laser au niveau du plan pupille de la sortie laser 6 est compris entre 4 mm et 8 mm ;
- un angle de faisceau au niveau de la sortie laser est inférieur à 5° ;
- la boucle de régulation comprend deux sous-boucles de régulation :
   - une première sous-boucle de régulation comprenant un premier imageur configuré pour recevoir une image de la rétine dérivée du chemin optique en aval du premier actionneur selon un premier champ d'acquisition,
   - une seconde sous-boucle de régulation comprenant un second imageur configuré pour recevoir une image de la rétine dérivée du chemin optique en amont de l'optique adaptative selon un second champ d'acquisition.
- le premier champ présente un angle de champ au moins deux fois supérieur à un angle de champ du second champ, et/ou la seconde sous-boucle de régulation fonctionne selon une fréquence supérieure à une fréquence de la première sous-boucle ;
- le système comprend en outre un sous-système de visualisation 15 configuré pour recevoir une image de la rétine dérivée du chemin optique en aval du premier actionneur et acquérir des images selon le premier champ ;
- le système comprend en outre un second actionneur placé en amont de l'optique adaptative, configuré pour recevoir une commande de déplacement laser, et pour agir sur le trajet du faisceau laser pour déplacer l'impact laser dans trois directions distinctes de l'espace ;
- le système est configuré pour que le second actionneur déplace le trajet du faisceau laser pendant que le laser de photocoagulation émet le faisceau laser, de sorte à ce que l'impact laser sur la rétine se déplace entre plusieurs emplacements de façon continue ;
- le second actionneur comprend :
   - un scanner positionné dans le chemin optique et adapté pour réaliser un déplacement du faisceau laser dans un plan focal perpendiculaire à la propagation du faisceau laser, et
   - un connecteur positionné dans le chemin optique et mobile en translation contrôlée dans la direction du faisceau laser ;
- l'imageur comprend une caméra configurée pour acquérir une image de la rétine dérivée du chemin optique en amont de l'optique adaptative, la caméra étant mobile en translation contrôlée dans la direction de propagation de la lumière que reçoit ladite caméra ;
- l'optique adaptative comprend :
   - un élément correcteur, disposé sur le chemin optique,
   - un module de traitement configuré pour commander l'élément correcteur en fonction d'une analyse d'une surface d'onde de la lumière transitant sur le chemin optique ;
- l'analyse de la surface d'onde est fournie à partir d'un analyseur de surface d'onde configuré pour recevoir de la lumière du chemin optique en amont de l'élément correcteur et pour déterminer une mesure représentative d'une forme d'une surface d'onde de la lumière transitant le long du chemin optique, ou l'imageur comprend une caméra configurée pour acquérir une image de la rétine dérivée du chemin optique en amont de l'optique adaptative, ou l'analyse de la surface d'onde est fournie à partir d'une image acquise par l'imageur comprenant une caméra configurée pour acquérir une image de la rétine dérivée du chemin optique en amont de l'optique adaptative ;
- le système comprend un dispositif de tomographie en cohérence optique relié au chemin optique en aval de l'optique adaptative ;
- la source laser est une source laser monomode à fibre.

### PRESENTATION DES FIGURES

L'invention sera mieux comprise, grâce à la description ci-après, qui se rapporte à des modes de réalisations et des variantes selon la présente invention, donnés à titre d'exemples non limitatifs et expliqués avec référence aux dessins schématiques annexés, dans lesquels :
- la figure 1 illustre schématiquement les principaux composants d'un système de photocoagulation laser d'une rétine selon un mode de réalisation ;
- la figure 2 illustre une configuration conforme à celle illustrée par la figure 1.

### DESCRIPTION DETAILLEE

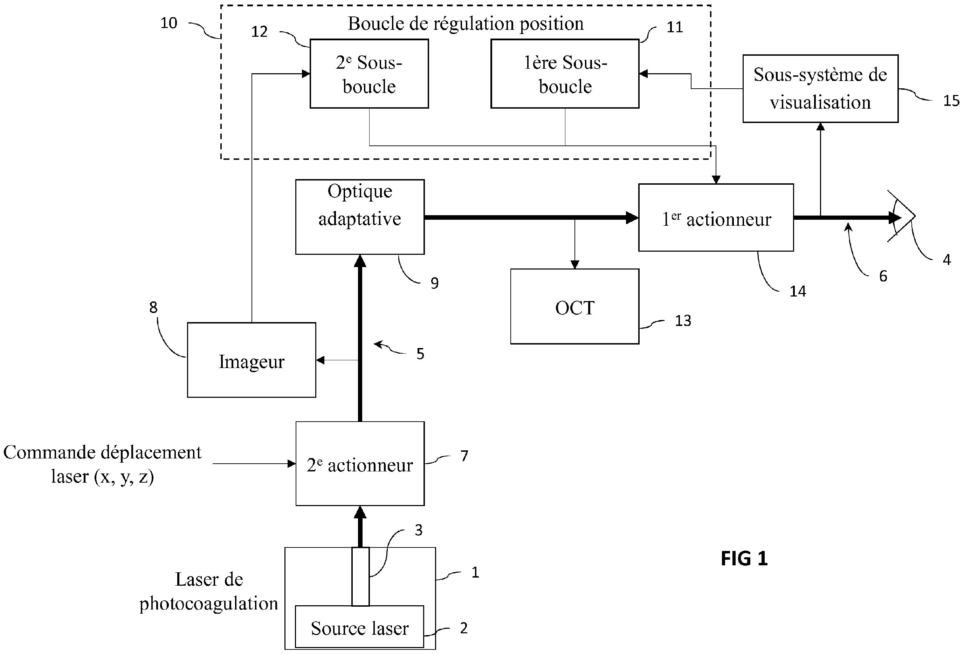

En référence à la figure 1 qui représente schématiquement les principaux éléments fonctionnels du système, le système de photocoagulation laser d'une rétine comprend un laser de photocoagulation 1 comprenant une source laser 2 et une fibre optique 3 de transport. La source laser 2 est de préférence une source laser à fibre, c'est-à-dire dont le milieu amplificateur est créé dans une fibre optique dopée par une terre rare. Cette fibre optique présente un diamètre de cœur de préférence inférieur à 12 µm, et de préférence encore inférieur à 8 µm, comme par exemple 6 µm. La source laser 2 est dite monomode, c'est-à-dire présentant un paramètre M² inférieur à 1,5, et de préférence inférieur à 1,2. L'aspect monomode et le faible diamètre de la fibre optique de la source laser 2 permet d'obtenir une faible perte de couplage (inférieure à 30%) lors de la transmission du faisceau laser dans la fibre optique de transport 3 du laser de photocoagulation 1.

La source laser 2 est configurée pour émettre dans une longueur d'onde comprise entre 520 nm et 690 nm dans la fibre optique de transport 3, et de préférence dans une longueur d'onde comprise entre 540 nm et 630 nm, et de préférence encore entre 550 nm et 600 nm. La source laser 2 est configurée pour émettre un faisceau laser présentant une puissance suffisante pour provoquer une photocoagulation de la zone ciblée de la rétine 4, et notamment pour provoquer l'effet thermique requis. Par exemple, le faisceau laser peut présenter une puissance comprise entre 50 mW et 3000 mW.

La fibre optique de transport 3 reçoit le faisceau laser émis par la source laser 2 et permet à ce faisceau laser de se propager de façon non rectiligne sur une grande distance sans risque. La fibre optique de transport 3 permet ainsi un découplage spatial entre le laser de photocoagulation 1 et le reste du système, et permet donc de déporter la source laser à plusieurs mètres de la rétine 4. La fibre optique de transport 3 présente un diamètre de cœur inférieur à 50 µm, et de préférence inférieur à 25 µm, de préférence encore inférieur à 15 µm, comme par exemple 12,5 µm. De ce fait, cette fibre de transport 3 peut être monomode ou légèrement multimode spatialement. De préférence, la fibre de transport 3 présente un paramètre M² inférieur à 1,5, et de préférence inférieur à 1,2.

En outre, c'est la sortie de cette fibre optique de transport 3 du laser de photocoagulation 1 qui est imagée à travers le reste du système sur la rétine du patient. Le faible diamètre de la fibre optique de transport 3 se traduit par un point focal de faible dimension, améliorant le confinement de l'impact laser. Ainsi, le système se caractérise par un diamètre du faisceau laser au niveau du plan pupille (c'est-à-dire à la position où la pupille du patient est positionnée) de la sortie laser 6 compris entre 4 mm et 8 mm. De préférence, le système se caractérise également par une faible ouverture numérique au niveau de la sortie laser 6. L'ouverture numérique dans l'air correspond au sinus du demi-angle d'ouverture, qui est l'angle entre l'axe optique et le rayon le plus écarté de l'axe optique, également appelé angle du faisceau. De préférence, cet angle de faisceau est inférieur à 5°, de préférence 2,5°, de préférence encore 0,5°, voire de préférence encore inférieur à 0,1°.

En présence d'un œil, le faisceau laser incident est focalisé par le cristallin de l'œil vers la rétine, qui constitue le plan focal. Le cristallin agit comme une lentille faisant converger les rayons incidents vers la rétine. Cette convergence est d'autant meilleure que les rayons incidents sont parallèles à l'axe optique. Une faible ouverture numérique au niveau de la sortie laser 6 se traduit par un faisceau laser à l'entrée de l'œil, et donc incident au cristallin, pratiquement collimaté, et donc par une focalisation quasi-ponctuelle au niveau de la rétine. A l'inverse, une forte ouverture numérique au niveau de la sortie laser 6 se traduirait par une focalisation imparfaite par le cristallin, conduisant à une tache étendue.

En outre, à l'intérieur de l'œil, le faisceau laser qui a traversé le cristallin peut également être caractérisé par une ouverture numérique oculaire lors de sa convergence vers la rétine. Cette ouverture numérique oculaire dépend notamment du diamètre du faisceau et de l'inverse de la focale de l'œil. Avec un diamètre du faisceau laser au niveau du plan pupille de la sortie laser 6 compris entre 4 mm et 8 mm, au lieu d'environ 1 mm pour les systèmes de l'état de la technique, on assure une grande ouverture numérique oculaire. En outre, le fait que le faisceau laser incident sur la pupille soit collimaté assure une bonne focalisation sur la rétine.

Or, la résolution latérale (perpendiculaire à la direction de propagation du faisceau, en x, y) de l'impact laser défini par la tache focale sur la rétine est proportionnelle (pour un œil parfait à ouverture circulaire) à l'inverse de l'ouverture numérique oculaire, tandis que l'extension en profondeur est proportionnelle au carré de l'inverse de l'ouverture numérique oculaire. Il en résulte que la forte ouverture numérique oculaire permet de limiter la dispersion du faisceau laser au niveau de la tache focale sur la rétine, et donc de confiner l'impact laser. En outre, tant la résolution latérale que l'extension en profondeur sont proportionnelles à la longueur d'onde. Le choix d'une longueur d'onde basse (inférieure à 690 nm, de préférence inférieure à 650 nm, et de préférence encore inférieure à 600 nm) permet d'améliorer encore ce confinement.

Or, ce faible confinement de l'impact nécessite de pallier les défauts des systèmes de l'état de la technique, qui étaient masqués par la grande taille de leur tache focale, et en particulier la nécessité de compenser les aberrations oculaires pour le laser, d'améliorer la précision de la localisation, et de permettre une visualisation fine de l'emplacement de l'impact laser tant en extension latérale qu'en profondeur. Pour ce faire, le système présente un chemin optique 5 pourvu de différents éléments qui vont maintenant être décrits. De façon générale, le faisceau laser émis par le laser de photocoagulation 1 se propage dans un chemin optique 5 défini par le système et reliant le laser de photocoagulation 1 en amont à une sortie laser 6 en aval, destinée à être placée face à la rétine 4.

Sur ce chemin optique 5 se trouvent :
- une optique adaptative 9 positionnée dans le chemin optique 5 et configurée pour modifier le front d'onde du faisceau laser se propageant dans le chemin optique 5 afin de compenser des aberrations oculaires se produisant jusqu'à la rétine 4,
- une boucle de régulation en position 10 commandant un premier actionneur 14 positionné dans le chemin optique en aval de l'optique adaptative pour asservir en position la sortie laser 6 vis-à-vis de la rétine 4 à traiter,
- au moins un imageur 8 configuré pour acquérir une image de la rétine 4 dérivée du chemin optique 5.

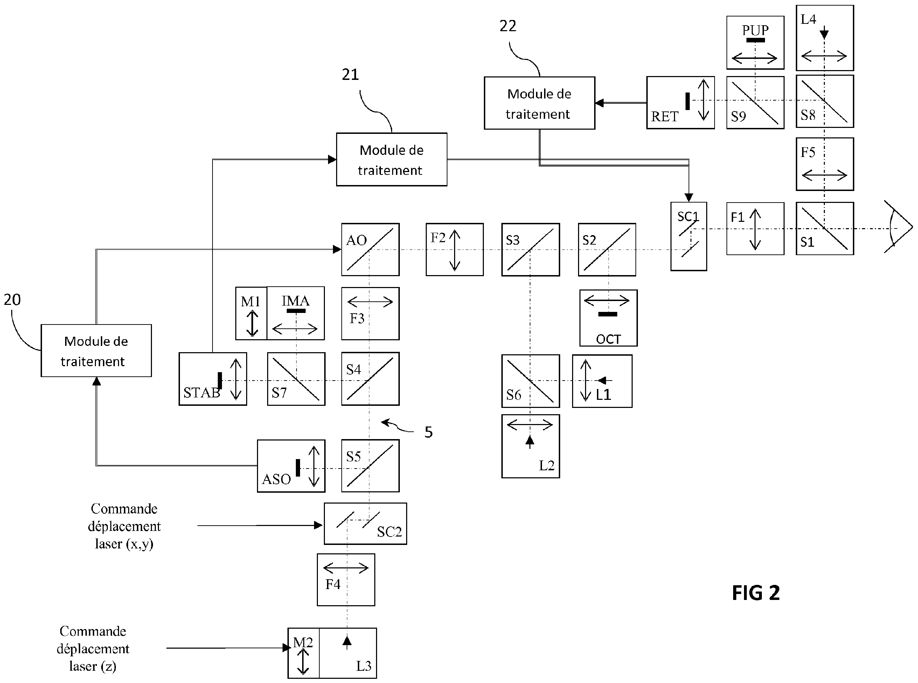

En référence à la figure 2, un exemple illustratif est décrit ci-dessous, dont les différents éléments peuvent intervenir dans différentes configurations au choix, qui sont décrits depuis l'aval du chemin optique vers l'amont. Il est bien entendu que diverses modifications pourraient être apportées à l'agencement des éléments constitutifs du système. Le système comprend une sortie laser 6 en aval, destinée à être placée face à la rétine 4. Une première séparatrice S1 sur le chemin optique 5 permet de dériver du chemin optique une image de la rétine 4. Dans la description, une séparatrice, ou "beam splitter" en anglais, permet de laisser passer une partie d'un rayon lumineux incident tout en réfléchissant une autre partie dans une autre direction, et peut être de tout type, comme par exemple un miroir semi-réfléchissant.

En amont de cette première séparatrice S1 se trouve une lentille de mise en forme F1, permettant notamment de réduire l'angle de champ, par exemple à des valeurs comprises entre 15° et 25°, en choisissant la longueur focale équivalente appropriée. Un scanner SC1 constituant le premier actionneur 14 permet de commander le déplacement du faisceau lumineux dans le chemin optique par rapport à la rétine 4. Le premier actionneur 14 permet de balayer toute la zone centrale de la rétine, notamment en raison de l'angle de champ qui est encore assez grand.

En amont du premier actionneur 14 formé par le premier scanner SC1, le chemin optique 5 comporte une séparatrice S2 qui permet de dériver une image de la rétine 4 vers un dispositif de tomographie en cohérence optique OCT, qui est donc relié au chemin optique 5 par cette séparatrice S2. Sur le chemin optique 5 se trouve également une séparatrice S3 permettant de faire pénétrer dans le chemin optique 5 de la lumière d'éclairage. Après une autre lentille de mise en forme F2 se trouve un élément correcteur AO de l'optique adaptative 9.

Le chemin optique 5 comporte ensuite vers l'amont une lentille F3 de mise en forme, puis une séparatrice S4 utilisée pour dériver du chemin optique 5 une image de la rétine vers un ou plusieurs imageurs, en amont de l'optique adaptative 9. Une autre séparatrice S5 sur le chemin optique S5 permet de dériver de la lumière du chemin optique 5 vers un analyseur de surface d'onde ASO.

Le chemin optique 5 comprend ensuite un second actionneur 7 placé en amont de l'optique adaptative, configuré pour recevoir une commande de déplacement laser, et pour agir sur le faisceau laser pour déplacer l'impact laser dans trois directions distinctes de l'espace. Plus précisément, il s'agit d'un actionneur 7 de balayage tridimensionnel du laser, permettant de déplacer l'impact laser au niveau de la rétine 4 au moins dans la direction de propagation du laser, notée z, mais également dans un plan perpendiculaire à la direction de propagation du laser, défini par deux directions distinctes notées x et y.

Pour ce faire, le second actionneur 7 comprend, ainsi qu'illustré sur la figure 2, un scanner SC2 positionné dans le chemin optique et adapté pour réaliser un déplacement du faisceau laser dans un plan focal (x, y) perpendiculaire à la propagation du faisceau laser, et un connecteur L3 positionné dans le chemin optique 5 et mobile en translation contrôlée dans la direction du faisceau laser. Le connecteur L3 fait la jonction entre la fibre optique de transport 3 et le chemin optique 5. Le faisceau laser émis par le laser de photocoagulation 1 passe de la fibre optique de transport 3 au chemin optique 5 par ce connecteur L3, qui peut donc être considéré comme une source pour les éléments en aval. Le scanner SC2 agit par exemple en modifiant les orientations respectives de deux miroirs se faisant face, décalant ainsi angulairement le faisceau laser qui en ressort.

Le connecteur L3 est monté sur une platine motorisée M2 recevant une commande de déplacement laser et se déplaçant en fonction de cette commande. Le déplacement de du connecteur L3 le long du chemin optique 5 entraîne une modification de son plan de focalisation, qui se répercute le long du chemin optique 5 pour aboutir à déplacer le plan focal à la sortie laser 6, c'est-à-dire le foyer de la rétine à traiter, dans la direction de propagation du faisceau laser. L'utilisateur peut ainsi modifier finement la profondeur traitée par l'impact laser, et par exemple modifier cette profondeur lorsqu'il est constaté que l'impact laser toucherait des tissus fonctionnels. Ce réglage fin en profondeur est d'autant plus utile qu'en raison du fort confinement de l'impact laser obtenu grâce au système, la profondeur d'un impact laser ne couvre plus nécessairement toute la zone à traiter, et qu'il peut être nécessaire de procéder à deux impacts laser à des profondeurs différentes pour traiter l'ensemble de la zone à traiter.

Dans le cas illustré par la figure 2, le scanner SC2 est placé en aval du connecteur L3. Il serait cependant possible d'intervertir leur ordre. Par ailleurs, la figure 2 montre la présence d'une lentille F4 qui est présente pour la mise en forme du faisceau laser à la sortie du connecteur L3. Son diamètre et sa distance focale sont choisies pour permettre le trajet du faisceau laser de façon satisfaisante entre les éléments constitutifs du système. La disposition comme les caractéristiques de cette lentille F4 sont donc laissés à l'appréciation de l'homme du métier, comme le sont celles des autres lentilles F1, F2 et F3.

L'optique adaptative 9 est formée de plusieurs éléments. Ainsi qu'évoqué plus haut, l'optique adaptative 9 comprend un élément correcteur AO, utilisé pour corriger le front d'onde du faisceau laser. Cette correction à apporter au front d'onde est déterminé au moyen d'un capteur placé en amont de l'élément correcteur AO, et configuré pour recevoir de la lumière du chemin optique 5 dont la nature dépend de la configuration choisie.

Dans l'exemple illustré par la figure 2, la configuration de l'optique adaptative 9 est dite "Shack-hartmann", et comprend un analyseur de surface d'onde ASO, ou analyseur de front d'onde, configuré pour recevoir de la lumière du chemin optique 5. Dans le cas illustré, la lumière est dérivée au moyen de la séparatrice S5 placée sur le chemin optique 5. L'analyseur de surface d'onde ASO détermine une mesure représentative d'une forme d'une surface d'onde de la lumière transitant le long du chemin optique 5. Plus précisément, l'analyseur de surface d'onde ASO décompose un front d'onde en fronts d'ondes élémentaires et il permet de déterminer pour chaque front d'onde élémentaire son orientation. La mesure de ces orientations permet après intégration de remonter à la forme du front d'onde.

D'autres configurations sont possibles. Notamment, il est possible de prévoir une optique adaptative sans capteur dédié, en exploitant par exemple l'imageur 8 configuré pour acquérir une image de la rétine 4 dérivée du chemin optique 5. L'analyse de la surface d'onde est alors faite directement sur l'image obtenue par cet imageur 8.

Ainsi qu'expliqué plus haut, la propagation de la lumière dans l'œil met en évidence des aberrations oculaires qui font naître des défauts dans cette lumière, et en particulier en ce qui concerne son front d'onde. Pour mieux détecter ces altérations du front d'onde, une source lumineuse L1, associée à une lentille, émet une lumière dont le faisceau est assimilable à celui émis par une source ponctuelle, de faible étendue. De préférence, cette lumière présente des longueurs d'onde comprise entre 600 nm et 700 nm, notamment car cette plage met en valeur les aberrations principales, et est de préférence encore monochromatique. Cette lumière est renvoyée par une séparatrice S6 vers le chemin optique 5, plus précisément vers la séparatrice S3 déjà évoquée, et atteint l'œil par la sortie laser 6. Une source ponctuelle artificielle est ainsi crée sur la rétine 4, qui réémet par diffusion dans toutes les directions. Le flux lumineux rétrodiffusé se propage à travers l'œil, puis le chemin optique 5 jusqu'à l'analyseur ASO, qui l'analyse dans un plan pupille et en détermine les déformations de front d'onde, représentatives des aberrations oculaires.

Les mesures de l'analyseur de surface d'onde ASO sont reçues et traitées par un module de traitement 20, de préférence un calculateur temps réel, configuré pour recevoir la mesure de l'analyseur de surface d'onde, et commander l'élément correcteur AO en fonction de la mesure de l'analyseur de surface d'onde, afin de compenser les perturbations détectées. Dans le cas typique où l'élément correcteur AO est un miroir déformable, le module de traitement 20 calcule les commandes (par exemple tensions ou intensités) à envoyer au miroir déformable, également placé dans un plan pupille. La surface du miroir déformable se modifie alors pour compenser les déformations de front d'onde mesurées.

L'action de l'élément correcteur permet donc de compenser les déformations pour tous les flux lumineux en provenance de la rétine 4, comme par exemple ceux à destinations d'imageurs placés en amont de l'élément correcteur, mais permet également de pré-modifier les flux lumineux provenant de l'amont de l'élément correcteur à destination de la rétine 4. Ces flux lumineux pré-modifiés présentent alors des déformations inverses de celles qu'ils subissent lors de leur trajet vers la rétine 4, de sorte qu'en atteignant la rétine 4, les aberrations oculaires sont compensées. Par conséquent, la lumière issue du laser de photocoagulation 1 présente, au niveau de l'impact laser sur la rétine 4, une qualité et une précision très améliorée. En particulier, le confinement de l'impact laser est amélioré.

La correction apportée par l'optique adaptative profite également aux imageurs disposés en amont de l'élément correcteur AO. De ce fait, le système comprend également un imageur 8 configuré pour acquérir une image de la rétine 4 dérivée du chemin optique 5, en amont de l'optique adaptative 9. De préférence, l'imageur 8 est mobile en translation contrôlée dans la direction de propagation de la lumière que reçoit l'imageur 8. Pour ce faire, l'imageur 8 comprend une caméra d'imagerie IMA montée sur une platine motorisée M1 qui permet, en fonction de commandes reçues, de déplacer cette caméra d'imagerie IMA. La séparatrice S4 permet de dériver une partie du flux lumineux parcourant le chemin optique 5 vers la caméra d'imagerie IMA, ce qui permet d'obtenir une image bidimensionnelle de la zone de la rétine faisant face à la sortie 6 à une profondeur de focalisation donnée. Ainsi qu'évoqué plus haut, la caméra d'imagerie IMA peut être utilisée pour l'analyse de la surface d'onde permettant de commander l'élément correcteur AO de l'optique adaptative 9 dans une configuration sans analyseur de front d'onde dédié.

Au moyen de la platine motorisée M1, la caméra d'imagerie IMA peut être déplacée pour modifier la profondeur de focalisation au niveau de la rétine 4. Il est alors possible d'imager la rétine à des profondeurs différentes. De fait, en raison du confinement important du laser obtenu grâce au système, la profondeur d'un impact laser ne couvre plus nécessairement toute la zone à traiter. La seule information de l'emplacement de l'impact laser n'est plus suffisante. Il est alors préférable de pouvoir imager à différentes profondeurs afin que l'utilisateur puisse visualiser l'étendue en profondeur de la zone à traiter, ou de la zone déjà traitée.

L'imageur 8 peut comprendre, à la place de la caméra d'imagerie IMA ou en complément, une caméra STAB, dite de stabilisation, pour acquérir une image de la rétine 4 dérivée du chemin optique 5, en amont de l'optique adaptative 9. Cette caméra de stabilisation STAB est utilisée pour mettre en œuvre une régulation pour asservir en position la sortie laser 6 vis-à-vis de la rétine 4 à traiter. Dans l'exemple illustré par la figure 2, l'imageur 8 comprend une caméra de stabilisation STAB et une caméra d'imagerie IMA. Une séparatrice S7 recevant du chemin optique 5 une partie du flux lumineux y circulant grâce à la séparatrice S4 permet d'alimenter en lumière ces deux caméras. L'imageur 8 est très en amont du chemin optique, et requiert une grande précision. A ce stade, l'angle de champ peut avoir été réduit par les différentes lentilles traversées F1, F2, F3 à une valeur inférieure à 5°.

Une source lumineuse L2 est prévue pour l'éclairage de la rétine pour l'imageur 8, c'est-à-dire ici pour la caméra de stabilisation STAB et/ou la caméra d'imagerie IMA. Cette source lumineuse L2 émet de préférence dans une longueur d'onde comprise entre 800 nm et 900 nm. La lumière émise par cette source lumineuse passe par la séparatrice S6 qu'elle partage avec la source lumineuse L1, puis rejoint le chemin optique 5 par la séparatrice S3. La lumière émise rejoint la rétine 4, et la lumière issue de la rétine 4 éclairée remonte le chemin optique 5 via l'optique adaptative 9. Les images acquises par l'imageur 8 bénéficient ainsi de la correction apportée par l'optique adaptative 9.

L'imageur 8 en amont de l'optique adaptative 9 est donc utilisé pour mettre en œuvre une régulation en position de la sortie laser 6 vis-à-vis de la rétine 4. Plus précisément, c'est la sortie de la caméra de stabilisation STAB qui est utilisée. La caméra STAB transmet des images acquises à un module de traitement 21, de préférence un calculateur temps réel, qui à partir de ces images détermine une commande du premier actionneur 14 pour déplacer le faisceau lumineux dans le chemin optique par rapport à la rétine 4 afin de stabiliser la position du faisceau lumineux, notamment en compensant les mouvements involontaires de l'œil. Le module de traitement 21 peut par exemple exploiter une image des photorécepteurs et un algorithme de type corrélation pour déterminer les mouvements de la rétine 4, et déterminer une commande du premier actionneur 14 pour que le faisceau lumineux suive ces mouvements.

Ainsi, le premier actionneur 14 est commandé par la boucle de régulation 10 pour asservir en position la sortie laser 6 vis-à-vis de la rétine 4 à traiter. Toutefois, une autre mesure peut être utilisée pour commander le premier actionneur 14. De préférence, la boucle de régulation 10 détermine une commande du premier actionneur 14 en fonction d'une part d'une première mesure dérivée du chemin optique 5 en aval du premier actionneur 14 et d'une seconde mesure dérivée du chemin optique 5 en amont du premier actionneur 14. Si cette seconde mesure est issue de l'imageur 8, la première mesure est issue d'un sous-système de visualisation grand champ, en aval du premier actionneur 14.

La première séparatrice S1, placée en aval du chemin optique 4, permet d'obtenir une visualisation large de la rétine 4, exploité par un sous-système de visualisation 15 grand champ. L'angle de champ peut alors typiquement être supérieur à 30°. Le sous-système de visualisation 15 peut être utilisé à la fois pour acquérir des images à destination de l'utilisateur, pour asservir en position le premier actionneur 14, et pour projeter sur la rétine une mire de fixation pouvant servir de repère visuel.

Ce sous-système de visualisation 15 comprend un ensemble source lumineuse et lentille L4 émettant en large bande de longueur d'onde, typiquement sur une majeure partie du spectre visible. Le flux lumineux ainsi émis rejoint le chemin optique 5 par la première séparatrice S1, via une autre séparatrice S8 et une lentille de mise en forme F5. Ce flux lumineux éclaire alors la rétine 4 afin de permettre d'imager la rétine 4. Dans l'autre sens, le flux lumineux issu de la rétine 4 est dérivé du chemin optique 5 par la première séparatrice S1, passe dans la lentille de mise en forme F5, et est déviée par la séparatrice S8 en direction des imageurs de visualisation. Une autre séparatrice S9 permet de diriger le flux vers deux imageurs de visualisation : un imageur de pupille PUP, qui permet d'acquérir et d'afficher l'image de la pupille, et un imageur de rétine RET, qui permet d'acquérir et d'afficher l'image de la rétine. L'imageur de rétine RET permet de mettre en œuvre la régulation de position du scanner SC1 faisant office de premier actionneur 14.

Ainsi, la boucle de régulation 10 comprend deux sous-boucles de régulation. La première sous-boucle de régulation 11 comprend un premier imageur configuré pour recevoir une image de la rétine dérivée du chemin optique en aval du premier actionneur 14 selon un premier champ d'acquisition. Ce premier imageur est l'imageur rétine RET, et le premier champ d'acquisition est donc le champ d'angle supérieur à 30°. Les images acquises par l'imageur rétine RET sont transmises à un module de traitement 22, typiquement un calculateur temps-réel, qui détermine une commande pour le premier scanner SC1.

Une seconde sous-boucle de régulation 12 comprend l'imageur 8 configuré pour recevoir une image de la rétine dérivée du chemin optique en amont de l'optique adaptative 9 selon un second champ d'acquisition. Il s'agit de la sous-boucle de régulation comprenant la caméra de stabilisation STAB et le module de traitement 21. De préférence, le premier champ présente un angle de champ au moins deux fois supérieur à un angle de champ du second champ, et de préférence supérieur à 10°. Par exemple, si le premier champ d'acquisition est le champ d'angle supérieur à 30°, le second champ d'acquisition présente un angle inférieur à 15°, et de préférence inférieur à 10°. Comme le champ d'acquisition est défini par l'imageur utilisé, dans le cas présenté plus haut, le second champ d'acquisition présente un angle inférieur à 5°, comme celui de l'imageur 8.

De préférence, également, la seconde sous-boucle de régulation 12 fonctionne selon une fréquence supérieure à la fréquence de la première sous-boucle 11. Par exemple, la première sous-boucle 11 peut fonctionner avec une fréquence inférieure à 100 Hz, voire inférieure à 75 Hz, par exemple de 60 Hz, tandis que la seconde sous-boucle 12 peut fonctionner avec une fréquence supérieure à 150 Hz, par exemple 200 Hz. On peut ainsi désigner la première sous-boucle 11 comme une boucle grand champ ou basse fréquence, et la seconde sous-boucle 12 comme une boucle petit champ ou haute fréquence.

Il est à noter que les différents modules de commande 20, 21, 22 peuvent être combinés entre eux ou deux-à-deux, et il est possible par exemple que leurs fonctions respectives soient remplies par un seul calculateur, de préférence temps-réel. Par ailleurs, le système peut comprendre un écran de visualisation vers lequel sont envoyés les images acquises par les imageurs tels que l'imageur PUP, la caméra IMA ou le dispositif de tomographie en cohérence optique OCT, afin que l'utilisateur du système puisse les visualiser.

De préférence, le dispositif de tomographie en cohérence optique OCT est synchronisé avec d'autres imageurs afin de faire correspondre les images acquises par le dispositif de tomographie en cohérence optique OCT avec les images acquises par un autre imageur. Cela signifie que des paramètres d'acquisition du dispositif de tomographie en cohérence optique OCT sont liés à des paramètres d'acquisition d'autres imageurs. De préférence, l'image acquise par le dispositif de tomographie en cohérence optique OCT est synchronisée sur l'image de la rétine obtenue par les autres imageurs. Cela permet à l'utilisateur de pouvoir visualiser la même zone sur l'image OCT et sur les autres images. Cela permet également de connaître la position du tir laser sur l'image OCT.

De préférence également, le dispositif de tomographie en cohérence optique OCT est configuré pour acquérir une image OCT pendant l'émission du faisceau laser par le laser de photocoagulation 1 de sorte que l'utilisateur puisse visualiser en temps réel l'effet du faisceau laser sur la rétine 4. Les différents imageurs permettent bien entendu également une visualisation après et avant l'impact laser. Grâce au système décrit ci-dessus, la taille de l'impact laser reste petit, étant plus confiné que les systèmes antérieurs, et l'utilisateur peut observer en trois dimensions et en temps-réel l'impact laser. Il est alors possible de mettre en œuvre un déplacement du faisceau laser sans interrompre celui-ci, afin de couvrir une zone à traiter, à l'inverse des systèmes antérieurs dans lesquels plusieurs impacts laser distincts étaient réalisés, le point d'impact laser étant déplacé entre deux émissions du faisceau laser. Il est également possible que le système soit configuré pour émettre le faisceau laser de façon discontinue, mais dont la discontinuité ne soit pas liée au déplacement du faisceau. De préférence, le système est configuré pour que le second actionneur 7 déplace le trajet du faisceau laser pendant que le laser de photocoagulation 1 émet le faisceau laser, de sorte à ce que l'impact laser sur la rétine se déplace entre plusieurs emplacements de façon continue. Le déplacement du faisceau laser est de préférence réalisé par le second actionneur 7, mais pourrait alternativement ou en complément être réalisé par le premier actionneur 14.

L'invention est définie les revendications suivantes.

## Revendications

1. Système de photocoagulation laser d'une rétine comprenant :
- un laser de photocoagulation (1) comprenant une source laser (2) et une fibre optique de transport (3),
- un chemin optique (5) reliant le laser de photocoagulation (1) en amont à une sortie laser (6) en aval destinée à être placée face à la rétine (4), un faisceau laser émis par le laser de photocoagulation étant destiné à se propager le long du chemin optique (5),
dans lequel la source laser (1) est une source laser configurée pour émettre dans une longueur d'onde comprise entre 520 nm et 690 nm dans la fibre optique (3), et en ce que la fibre optique de transport (3) présente un cœur avec un diamètre inférieur à 50 µm,
le système **caractérisé en ce que** il comprend :
- une optique adaptative (9) positionnée dans le chemin optique (5) et configurée pour modifier le front d'onde du faisceau laser se propageant dans le chemin optique afin de compenser des aberrations oculaires se produisant jusqu'à la rétine (4),
- une boucle de régulation en position (10) commandant un premier actionneur (14) positionné dans le chemin optique (5) en aval de l'optique adaptative (9) pour asservir en position la sortie laser vis-à-vis de la rétine à traiter,
- au moins un imageur (8) configuré pour acquérir une image de la rétine dérivée du chemin optique (5).

2. Système selon la revendication 1, dans lequel un diamètre du faisceau laser au niveau du plan pupille de la sortie laser (6) est compris entre 4 mm et 8 mm.

3. Système selon l'une des revendications 1 ou 2, dans lequel un angle de faisceau au niveau de la sortie laser (6) est inférieur à 5°.

4. Système selon l'une des revendications précédentes, dans lequel la boucle de régulation (10) comprend deux sous-boucles de régulation :
- une première sous-boucle de régulation (11) comprenant un premier imageur (RET) configuré pour recevoir une image de la rétine dérivée du chemin optique (5) en aval du premier actionneur (14) selon un premier champ d'acquisition,
- une seconde sous-boucle de régulation (12) comprenant un second imageur (8) configuré pour recevoir une image de la rétine dérivée du chemin optique (5) en amont (8) de l'optique adaptative (9) selon un second champ d'acquisition.

5. Système selon la revendication précédente, dans lequel le premier champ présente un angle de champ au moins deux fois supérieur à un angle de champ du second champ, et/ou la seconde sous-boucle de régulation (12) fonctionne selon une fréquence supérieure à une fréquence de la première sous-boucle (10).

6. Système selon l'une des revendications 4 ou 5, comprenant en outre un sous-système de visualisation (15) configuré pour recevoir une image de la rétine dérivée du chemin optique (5) en aval du premier actionneur (14) et acquérir des images selon le premier champ.

7. Système selon l'une quelconque des revendications précédentes, comprenant en outre un second actionneur (7) placé en amont de l'optique adaptative (9), configuré pour recevoir une commande de déplacement laser, et pour agir sur le trajet du faisceau laser pour déplacer l'impact laser dans trois directions distinctes de l'espace.

8. Système selon la revendication 7, dans lequel le système est configuré pour que le second actionneur (7) déplace le trajet du faisceau laser pendant que le laser de photocoagulation (1) émet le faisceau laser, de sorte à ce que l'impact laser sur la rétine se déplace entre plusieurs emplacements de façon continue.

9. Système selon la revendication 7 ou la revendication 8, dans lequel le second actionneur (7) comprend :
- un scanner (SC2) positionné dans le chemin optique (5) et adapté pour réaliser un déplacement du faisceau laser dans un plan focal (x, y) perpendiculaire à la propagation du faisceau laser, et
- un connecteur (L3) positionné dans le chemin optique (5) et mobile en translation contrôlée dans la direction du faisceau laser.

10. Système selon l'une quelconque des revendications précédentes, dans lequel l'imageur (8) comprend une caméra (IMA) configurée pour acquérir une image de la rétine (4) dérivée du chemin optique en amont de l'optique adaptative (9), la caméra (IMA) étant mobile en translation contrôlée dans la direction de propagation de la lumière que reçoit ladite caméra (IMA).

11. Système selon l'une quelconque des revendications précédentes, dans lequel l'optique adaptative (9) comprend :
- un élément correcteur (AO), disposé sur le chemin optique (5),
- un module de traitement (20) configuré pour commander l'élément correcteur en fonction d'une analyse d'une surface d'onde de la lumière transitant sur le chemin optique.

12. Système selon la revendication précédente, dans lequel l'analyse de la surface d'onde est fournie à partir d'un analyseur de surface d'onde (ASO) configuré pour recevoir de la lumière du chemin optique (5) en amont de l'élément correcteur (AO) et pour déterminer une mesure représentative d'une forme d'une surface d'onde de la lumière transitant le long du chemin optique (5), ou l'imageur (8) comprend une caméra (IMA) configurée pour acquérir une image de la rétine (4) dérivée du chemin optique en amont de l'optique adaptative (9), ou l'analyse de la surface d'onde est fournie à partir d'une image acquise par l'imageur (8) comprenant une caméra (IMA) configurée pour acquérir une image de la rétine (4) dérivée du chemin optique en amont de l'optique adaptative (9).

13. Système selon l'une quelconque des revendications précédentes, comprenant un dispositif de tomographie en cohérence optique (OCT) relié au chemin optique en aval de l'optique adaptative (9).

14. Système selon l'une quelconque des revendications précédentes, dans lequel la source laser (1) est une source laser monomode à fibre.

## Patentansprüche

1. System zur Laserfotokoagulation einer Netzhaut, umfassend:
- einen Fotokoagulationslaser (1), der eine Laserquelle (2) und eine optische Transportfaser (3) umfasst,
- einen optischen Pfad (5), der den Fotokoagulationslaser (1) stromaufwärts mit einem stromabwärts gelegenen Laserausgang (6) verbindet, der dazu bestimmt ist, der Netzhaut (4) zugewandt angeordnet zu werden, wobei ein vom Fotokoagulationslaser emittierter Laserstrahl dazu bestimmt ist, sich entlang des optischen Pfads (5) auszubreiten,
wobei die Laserquelle (1) eine Laserquelle ist, die so konfiguriert ist, dass sie in einer Wellenlänge zwischen 520 nm und 690 nm in der optischen Faser (3) emittiert, und dass die optische Transportfaser (3) einen Kern mit einem Durchmesser von weniger als 50 µm aufweist,
wobei das System **dadurch gekennzeichnet ist, dass** es Folgendes umfasst:
- eine adaptive Optik (9), die im optischen Pfad (5) angeordnet und konfiguriert ist, um die Wellenfront des sich im optischen Pfad ausbreitenden Laserstrahls zu modifizieren, um okulare Aberrationen zu kompensieren, die bis zur Netzhaut (4) auftreten,
- eine Positionsregelungskreis (10), der einen ersten Aktuator (14) steuert, der im optischen Pfad (5) stromabwärts der adaptiven Optik (9) positioniert ist, um die Laserausgabe in Bezug auf die zu behandelnde Netzhaut in Position zu bringen,
- mindestens einen Bildgeber (8), der konfiguriert ist, um ein Bild der Netzhaut zu erfassen, das aus dem optischen Pfad (5) abgeleitet wird.

2. System nach Anspruch 1, wobei ein Durchmesser des Laserstrahls hinsichtlich der Pupillenebene des Laserausgangs (6) zwischen 4 mm und 8 mm liegt.

3. System nach einem der Ansprüche 1 oder 2, wobei ein Strahlwinkel hinsichtlich des Laserausgangs (6) kleiner als 5° ist.

4. System nach einem der vorhergehenden Ansprüche, wobei der Regelkreis (10) zwei Teilregelkreise umfasst:
- einen ersten Teilregelkreis (11), der einen ersten Bildgeber (RET) umfasst, der konfiguriert ist, um ein Bild der Netzhaut zu empfangen, das aus dem optischen Pfad (5) stromabwärts des ersten Aktuators (14) gemäß einem ersten Erfassungsfeld abgeleitet wird,
- einen zweiten Teilregelkreis (12), der einen zweiten Bildgeber (8) umfasst, der konfiguriert ist, um ein Bild der Netzhaut zu empfangen, das von dem optischen Pfad (5) stromaufwärts (8) der adaptiven Optik (9) gemäß einem zweiten Erfassungsfeld abgeleitet wird.

5. System nach dem vorhergehenden Anspruch, wobei das erste Feld einen Feldwinkel aufweist, der mindestens doppelt so groß ist wie ein Feldwinkel des zweiten Felds, und/oder der zweite Teilregelkreis (12) mit einer höheren Frequenz als eine Frequenz des ersten Teilregelkreises (10) arbeitet.

6. System nach einem der Ansprüche 4 oder 5, das ferner ein Visualisierungsteilsystem (15) umfasst, das konfiguriert ist, um ein Bild der Netzhaut zu empfangen, das von dem optischen Pfad (5) stromabwärts des ersten Aktuators (14) abgeleitet wird, und um Bilder gemäß dem ersten Feld zu erfassen.

7. System nach einem der vorhergehenden Ansprüche, das ferner einen zweiten Aktuator (7) umfasst, der stromaufwärts der adaptiven Optik (9) angeordnet ist und konfiguriert ist, um einen Laserbewegungsbefehl zu empfangen und auf den Pfad des Laserstrahls zu wirken, um die Laserwirkung in drei verschiedene Raumrichtungen zu bewegen.

8. System nach Anspruch 7, wobei das System so konfiguriert ist, dass der zweite Aktuator (7) den Weg des Laserstrahls bewegt, während der Fotokoagulationslaser (1) den Laserstrahl emittiert, so dass sich die Laserwirkung auf die Netzhaut kontinuierlich zwischen mehreren Stellen bewegt.

9. System nach Anspruch 7 oder Anspruch 8, wobei der zweite Aktuator (7) Folgendes umfasst:
- einen Scanner (SC2), der in dem optischen Pfad (5) positioniert und angepasst ist, um eine Bewegung des Laserstrahls in einer Fokusebene (x, y) senkrecht zur Ausbreitung des Laserstrahls auszuführen, und
- einen Verbinder (L3), der im optischen Pfad (5) positioniert und in gesteuerter Translation in Richtung des Laserstrahls bewegbar ist.

10. System nach einem der vorhergehenden Ansprüche, wobei der Bildgeber (8) eine Kamera (IMA) umfasst, die konfiguriert ist, um ein Bild der Netzhaut (4) zu erfassen, das von dem optischen Pfad stromaufwärts der adaptiven Optik (9) abgeleitet wird, wobei die Kamera (IMA) in der Ausbreitungsrichtung des von der Kamera (IMA) empfangenen Lichts in einer gesteuerten Translation bewegbar ist.

11. System nach einem der vorhergehenden Ansprüche, wobei die adaptive Optik (9) umfasst:
- ein Korrekturelement (AO), das im optischen Pfad (5) angeordnet ist,
- ein Verarbeitungsmodul (20), das so konfiguriert ist, um das Korrekturelement gemäß einer Analyse einer Wellenfront des Lichts zu steuern, das den optischen Pfad durchläuft.

12. System nach dem vorhergehenden Anspruch, wobei die Analyse der Wellenfront von einem Wellenfrontsensor (ASO) geliefert wird, der konfiguriert ist, das Licht des optischen Pfads (5) stromaufwärts des Korrekturelements (AO) zu empfangen und einen Messrepräsentanten einer Form einer Wellenfront des Lichts, das entlang des optischen Pfads (5) passiert, zu bestimmen, oder der Bildgeber (8) eine Kamera (IMA) umfasst, die konfiguriert ist, um ein Bild der Netzhaut (4) zu erfassen, das von dem optischen Pfad stromaufwärts von der adaptiven Optik (9) abgeleitet wird, oder die Analyse der Wellenfront aus einem Bild bereitgestellt wird, das von dem Bildgeber (8) erfasst wird, der eine Kamera (IMA) umfasst, die konfiguriert ist, um ein Bild der Netzhaut (4) zu erfassen, das von dem optischen Pfad stromaufwärts von der adaptiven Optik (9) abgeleitet wird.

13. System nach einem der vorhergehenden Ansprüche, umfassend eine optische Kohärenztomographie (OCT)-Vorrichtung, die im optischen Pfad stromabwärts der adaptiven Optik (9) angeschlossen ist.

14. System nach einem der vorhergehenden Ansprüche, wobei die Laserquelle (1) eine Monomodenfaser-Laserquelle ist.

## Claims

1. A system for laser photocoagulation of a retina comprising:
- a photocoagulation laser (1) comprising a laser source (2) and an optical transport fiber (3),
- an optical path (5) connecting the upstream photocoagulation laser (1) to a downstream laser output (6) intended to be placed in front of the retina (4), a laser beam emitted by the photocoagulation laser being intended to propagate along the optical path (5),
wherein the laser source (1) is a laser source configured to emit in a wavelength comprised between 520 nm and 690 nm in the optical fiber (3), and in that the optical transport fiber (3) has a core with a diameter less than 50 µm,
the system comprising:
- an adaptive optics (9) positioned in the optical path (5) and configured to modify the wavefront of the laser beam propagating in the optical path in order to compensate ocular aberrations occurring up to the retina (4),
- a position control loop (10) controlling a first actuator (14) positioned in the optical path (5) downstream of the adaptive optics (9) to servo-control the position of the laser output with respect to the retina to be treated,
- at least one imager (8) configured to acquire an image of the retina derived from the optical path (5).

2. The system according to claim 1, wherein a diameter of the laser beam at the pupil plane of the laser output (6) is comprised between 4 mm and 8 mm.

3. The system according to any of claims 1 or 2, wherein a beam angle at the laser output (6) is less than 5°.

4. The system according to any of the preceding claims, wherein the control loop (10) comprises two control sub-loops:
- a first control sub-loop (11) comprising a first imager (RET) configured to receive an image of the retina derived from the optical path (5) downstream of the first actuator (14) according to a first acquisition field,
- a second control sub-loop (12) comprising a second imager (8) configured to receive an image of the retina derived from the optical path (5) upstream (8) of the adaptive optics (9) according to a second acquisition field.

5. The system according to the preceding claim, wherein the first field has a field angle at least twice as great as a field angle of the second field, and/or the second control sub-loop (12) operates at a frequency greater than a frequency of the first sub-loop (10).

6. The system according to any of claims 4 or 5, further comprising a visualization subsystem (15) configured to receive an image of the retina derived from the optical path (5) downstream of the first actuator (14) and acquire images according to the first field.

7. The system according to any one of the preceding claims, further comprising a second actuator (7) placed upstream of the adaptive optics (9), configured to receive a laser displacement command, and to act on the path of the laser beam in order to move the laser impact in three distinct directions in space.

8. The system according to claim 7, wherein the system is configured so that the second actuator (7) moves the path of the laser beam while the photocoagulation laser (1) emits the laser beam, such that the laser impact on the retina moves between several locations in a continuous manner.

9. The system according to claim 7 or 8, wherein the second actuator (7) comprises:
- a scanner (SC2) positioned in the optical path (5) and adapted to perform a displacement of the laser beam in a focal plane (x, y) perpendicular to the propagation of the laser beam, and
- a connector (L3) positioned in the optical path (5) and movable in controlled translation in the direction of the laser beam.

10. The system according to any one of the preceding claims, wherein the imager (8) comprises a camera (IMA) configured to acquire an image of the retina (4) derived from the optical path upstream of the adaptive optics (9), the camera (IMA) being movable in controlled translation in the direction of propagation of the light received by said camera (IMA).

11. The system according to any one of the preceding claims, wherein the adaptive optics (9) comprises:
- a correcting element (AO), disposed on the optical path (5),
- a processing module (20) configured to control the correcting element based on an analysis of a wavefront of the light traveling over the optical path.

12. The system according to the preceding claim, wherein the analysis of the wavefront is provided from a wavefront sensor (ASO) configured to receive light from the optical path (5) upstream of the correcting element (AO) and to determine a measurement representative of a shape of a wavefront of the light traveling along the optical path (5), or the imager (8) comprises a camera (IMA) configured to acquire an image of the retina (4) derived from the optical path upstream of the adaptive optics (9), or the analysis of the wavefront is provided from an image acquired by the imager (8) comprising a camera (IMA) configured to acquire an image of the retina (4) derived from the optical path upstream of the adaptive optics (9).

13. The system according to any one of the preceding claims, comprising an optical coherence tomography (OCT) device connected to the optical path downstream of the adaptive optics (9).

14. The system according to any one of the preceding claims, wherein the laser source (1) is a single-mode fiber laser source.
